(19) Europäisches Patentamt
European Patent Office
Office européen des brevets

(11) Publication number: **0 668 288 A1**

(12) **EUROPEAN PATENT APPLICATION**

(21) Application number: **95102235.9**

(22) Date of filing: **17.02.95**

(51) Int. Cl.⁶: **C07H 1/06**, C12Q 1/54, //A61K49/00

(30) Priority: **17.02.94 JP 43115/94**

(43) Date of publication of application: **23.08.95 Bulletin 95/34**

(84) Designated Contracting States: **AT DE FR GB**

(71) Applicant: **Kureha Chemical Industry Co., Ltd.**
**9-11, Nihonbashihoridome-cho 1-Chome,**
**Chuo-ku**
**Tokyo 103 (JP)**

(72) Inventor: **Kikuchi, Tatsuji**
**4-28-20, Shimo, kita-ku**
**Todyo 115 (JP)**
Inventor: **Nitta, Toyohiko**
**6-18-14, Keyakidai, Morya-machi**
**Kitasoma-gun, Ibaraki (JP)**
Inventor: **Ito, Tomoko**
**2-8-12, Higashihorikiri, Ktsushika-ku**
**Tokyo 124 (JP)**

(74) Representative: **Cohausz & Florack**
**Patentanwälte**
**Kanzlerstrasse 8a**
**D-40472 Düsseldorf (DE)**

(54) **Method for assay of biological samples.**

(57) A method for the assay of inulin in a sample comprising the steps of:
(1) removing from the sample, glucose and fructose through a membrane; then
(2) treating the sample obtained from the above step 1 under acid-hydrolysis conditions capable of hydrolyzing inulin to fructoses; and then
(3) measuring the amount of fructoses in the sample obtained from the above step 2 is disclosed. According to the method, a disease lowering a kidney function can be diagnosed.

EP 0 668 288 A1

## BACKGROUND OF THE INVENTION

### 1. Field of the Invention

The present invention relates to a method for the assay of inulin.

### 2. Description of the Related Art

The glomerular filtration rate (GFR) is used as one of indicators of the kidney function. The GFR is determined by clearance tests such as creatinine clearance and inulin clearance.

Creatinine is inherently present in the body as a terminal metabolite of creatine, and so is broadly used as an indicator in simple methods for the assay of GFR, without administering any particular agents for the assay. There is, however, the defect that the measured value changes due to physiological fluctuations of the creatinine concentration in the blood and the reabsorption rate in the uriniferous tubule.

Inulin, however, is inactive sufficiently in a pharmacological standpoint of view; inulin does not bind with protein in the plasma, is completely filtered through the glomeruli in the kidney, but is not absorbed or secreted in the uriniferous tubule. Therefore, it is an ideal indicator for GFR determination. Accordingly, inulin clearance can be used to conduct most reliable GFR determination.

Inulin is a high molecular weight compound (polyfructosan) having a molecular weight of around 5000, but the molecular weight thereof is widely distributed. Thus, it is difficult to quantify inulin as it is.

Therefore, the method wherein inulin, polyfructosan, is converted to fructose by hydrolysis under an acidic condition, producing a color formation from the resulting fructose with one or more various coloring agents and performing a spectroscopic measurement, was used for a long time. As the color formation, the methods of treatment with diphenylamine [H.D. Harrison, Proc. Soc. Exp. Biol. Med., Vol. 49, pp. 111 - 114 (1942)], resorcin [G.F. Schreiner, Proc. Soc. Exp. Biol. Med., Vol. 74, pp. 117 - 120 (1950)], and anthrone [W.D. Davidson et al., J. Lab. Clin. Med., Vol. 62, pp. 351 - 356 (1963)] are known.

The above-mentioned method of spectroscopic measurement followed by acid-hydrolysis and color formation, however, was easily affected by the other components in the biological sample, non-specific, and inaccurate. The method wherein anthrone is used (anthrone method) is the most generally used of the above methods, but has the disadvantages in instability of the anthrone reagent, a low sensitivity to 5 mg/dl and thus a high optimum concentration of 25 mg/dl in the blood for the assay. Therefore, a large amount of inulin should be administered to maintain the concentration of inulin in the blood, and thus toxicity is concerned.

## SUMMARY OF THE INVENTION

The inventors of the present invention engaged in intensive studies to develop an assay which is not affected by other interferents in a sample to be tested and can be performed with less administration of inulin, and as a result discovered that it is possible to accurately determine inulin with high sensitivity by removing from the sample the low molecular weight saccharides such as glucose and fructose through a membrane, optionally removing proteins, acid-hydrolyzing the pretreated sample to convert inulin to fructose, and detecting the fructose produced.

Accordingly, an object of the present invention is to provide a means for accurately detecting inulin with high sensitivity.

Other objects and effects of the present invention will be clear from the following description.

The present invention relates to a method for the assay of inulin in a sample comprising the steps of:

(1) removing from the sample, glucose and fructose through a membrane; then

(2) treating the sample obtained from the above step 1 under acid-hydrolysis conditions capable of hydrolyzing inulin to fructose; and then

(3) measuring the amount of fructose in the sample obtained from the above step 2.

## BRIEF DESCRIPTION OF THE DRAWINGS

Figure 1 shows a calibration curve of inulin.

Figure 2 shows comparison of the results from the method for the assay of inulin according to the present invention with those from the conventional anthrone method.

Figure 3 shows comparison of the results from the method for the assay of inulin according to the present invention with those from the conventional enzyme method.

DESCRIPTION OF THE PREFERRED EMBODIMENTS

In the present invention, the sample means particularly a biological sample. As the biological sample, there may be mentioned plasma, serum or urine, or a dilution thereof with water, saline, physiological saline, or appropriate buffers. Further, the sample of the present invention also includes solutions prepared by dissolving inulin, glucose, fructose and/or protein in water, saline, physiological saline or buffer, as a model of the biological samples.

Inulin is a straight-chain fructan of the formula:

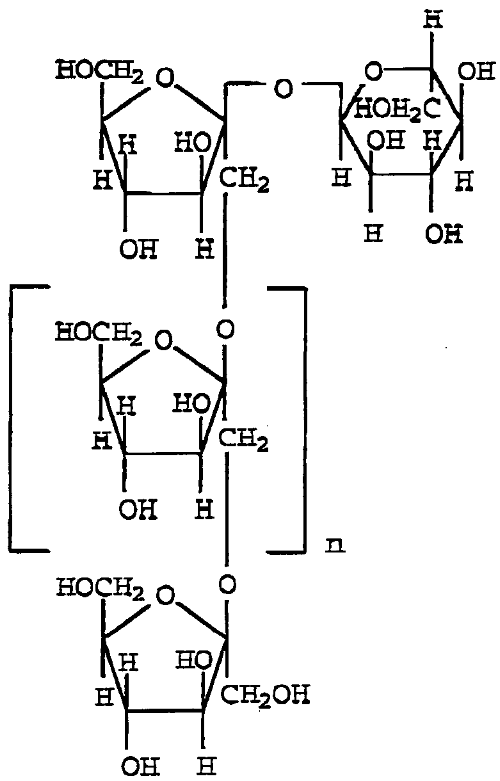

(I)

wherein the average of n is approximately 35. Inulin is composed of $\beta$-D-(2→1) fructose and has a non-reducible glucose at the terminus thereof. The molecular weight is approximately 5000. When inulin is hydrolyzed with an acid, D-fructose and D-glucose are obtained.

The steps of the method for the assay of inulin according to the present invention will be explained hereinafter.

Step 1: Removal of glucose and fructose from a sample through a membrane

In this step, glucose, fructose and other low molecular weight compounds are separated and removed from the sample through a membrane. The method of separation through the membrane is not particularly limited, so long as it is assured that inulin remains behind in the sample, but glucose, fructose and other low molecular weight compounds are separated and removed. For example, usual methods, such as precision filtration, ultrafiltration, reverse osmosis or dialysis may be used. As the separation through the membrane, dialysis is desirable in that accurate separation is possible even without adding or reducing pressure.

The procedure of dialysis is not especially limited and usual methods may be used, so long as the above requirements are satisfied. Preferably, the sample is sealed in a semipermeable membrane tube, placed in flowing water or saline solution and allowed to stand at 4 - 40 °C for 5 hours to 2 days. Further, the sample (0.1 - 2 ml) is placed in a flow dialyzer and dialyzed with water, saline solution or another dialysis solution (0.5 to 10 liters) at a circulation flow rate of 5 - 50 ml/min. The desirable volume of the dialysis solution is 25 - 50000 times the volume of the sample.

The semipermeable membrane used for the dialysis is not especially limited so long as a substance having the molecular weight of less than 1000, preferably less than 500 is removed. For example, a cellophane membrane, collodion membrane or cellulose derivative membrane may be used. As examples of a cellulose derivative membrane, there may be mentioned a cellulose ester (such as cellulose acetate) membrane and a regenerated cellulose membrane. If a semipermeable membrane rated to permeate substances of molecular weight of more than 1000 is used, inulin having a relatively low molecular weight may unfavorably pass through the membrane. If a semipermeable membrane rated to retain substances of molecular weight of less than 500, it is difficult to efficiently remove the glucose, fructose and/or low molecular weight compounds to be removed.

One of the main features of the method according to the present invention resides in the improvement of the sensitivity to detect fructose by removing the glucose, fructose and other low molecular weight saccharides inherently present in the sample through the membrane in the step 1.

More particularly, in the method of the present invention, inulin is hydrolyzed to fructose after the step 1 and then the fructose derived from the inulin is measured. When fructose is separated for example by high performance liquid chromatography and detected by a pulse electrochemical detector, a large amount of glucose inherently present in the sample must be removed in advance. If the glucose is not removed, the glucose is detected as large peak which is superposed on that of the fructose, and therefore, the sensitivity to fructose is remarkably reduced. The fructose inherently present in the sample must be removed in advance too, or else will be added to the peaks of the fructose derived from the inulin and produce an error. Further, the low molecular weight saccharides which may be present in the sample also may produce an error in the value of fructose measured.

When fructose is measured by an enzymatic method, a large amount of glucose present in the sample must also be removed in advance, or else will interfere with measurement of the fructose. Of course, the fructose inherently present in the sample must be removed in advance too, or else will produce an error in the measured value of fructose derived from the inulin, and the low molecular weight saccharides may also produce an error in the value of the fructose measured.

As the conventional methods using dialysis, there are known the method of taking out glucose in the blood by dialysis and measuring the concentration of glucose [Japanese Unexamined Patent Publication (Kokai) No. 52-85885)] and the method of separating inulin and glucose by dialysis of a small amount (100 µl) of a sample [P.H. Brand, et al., Anal. Biochem., Vol. 94, pp. 109 - 111 (1979)]. However, there is no conventional method wherein dialysis is applied to inulin clearance measurement.

Optional step: Removal of proteins from a sample

In the method of the present invention, the inulin is acid-hydrolyzed with a strong acid after the step 1. Therefore, when a protein is included in the sample, it is necessary to remove such a protein in advance. A plasma or serum sample derived from blood contains proteins, and thus a step for removing proteins is necessary. When the sample is derived from urine, it is not always essential to conduct the step for removing proteins. However, the step for removing proteins has to be performed for proteinuria.

If the step for hydrolyzing inulin to fructose is performed for the sample containing the remaining protein, the protein may be denatured or hydrolyzed in the hydrolysis step, whereby the liquid chromatography column is plugged, or interference peaks appear. Further, this can have an influence on the absorbance.

Further, if the step for removing the protein is performed before the above-mentioned step 1, the inulin may also be partially hydrolyzed under the acidic conditions for removing the protein, and the hydrolyzed products may be removed by the above-mentioned membrane separation. Thus, the value measured may include an error therefrom. Further, an osmotic pressure of the supernatant after removal of the protein considerably varies, and the preparation of the dialysis solution becomes complicated. Accordingly, the step for removing a protein is required to carry out between the above-mentioned step 1 and the subsequent step 2.

As the precipitating agent for a protein, an aqueous solution of trichloroacetic acid (TCA), perchloric acid, phenol, chloroform or chloroformmethanol solution may be used. It is preferable to use an aqueous solution of TCA, because the amount thereof to be added may be small, and thus, there is no adverse influence on the fructose detection. Further, it is preferable to use perchloric acid, because it is possible to perform the subsequent acid-hydrolysis of inulin without any additional acid. The concentration of the aqueous solution of TCA is preferably 2 to 15% by weight, more preferably 2 to 10% by weight. If the concentration of the aqueous solution of TCA is less than 2% by weight, the non-precipitated proteins remain behind in the sample, and if the concentration is more than 15% by weight, the inulin is included in

the denatured proteins to produce an error. Further, a final concentration of 0.1 to 2.0 N perchloric acid is preferable. If the final concentration of perchloric acid is less than 0.1 N, the non-precipitated proteins remain behind in the sample, and even if the final concentration is more than 2.0 N, the amount of the proteins removed cannot be further increased.

The step for removing the protein is performed by adding the precipitating agent to the sample at generally 4 to 40 °C, preferably 5 to 35 °C, more preferably room temperature to precipitate the protein, and then removing the proteins precipitated by centrifugal separation or filtration.

Step 2: Acid-hydrolysis of inulin in sample to fructose

In this step 2, the inulin is hydrolyzed and converted to fructose. As the acid, an inorganic acid, such as sulfuric, hydrochloric or perchloric acid may be used. The concentration of the acid used, the reaction temperature and the reaction time can be appropriately determined in view of the acid used. For example, sulfuric acid is preferably added to the sample free of proteins so that a final concentration is 0.5 to 2.0 N and the mixture is heated at 20 to 100 °C for 1 minute to 20 hours. When hydrochloric acid is used, the acid is preferably added to the sample free of proteins so that a final concentration is 0.5 to 2.0 N and the mixture is heated at 50 to 100 °C for 1 minute to 20 hours. In the case of perchloric acid, the acid is preferably added to the sample free of proteins so that a final concentration is 0.1 to 2.0 N and the mixture is heated at 20 to 100 °C for 1 minute to 20 hours. When the sample from urine is used and thus the step for removing a protein is not necessary, the sample from the step (1) may be directly used.

If the above final concentrations of the acids are lower than the above lower limits, sometimes the conversion of inulin to fructose becomes insufficient. When the above upper limit is exceeded, there is the possibility that the fructose per se will be decomposed. Furthermore, when the above reaction temperature and reaction time become lower than the above lower limits, sometimes the conversion of inulin to fructose becomes insufficient. When the above upper limits are exceeded, there is the possibility that the fructose per se will be decomposed.

When perchloric acid is used as the precipitating agent (preferable final concentration = 0.1 to 2.0N) in the above step for removing a protein, the perchloric acid contained in the sample free of protein may be used as it is, and the acid-hydrolysis can be performed by heating to the above-mentioned temperature.

The sample obtained by the hydrolysis may be used as it is for measuring the amount of fructose. However, the sample sometimes has an adverse effect on separation when liquid column chromatography is used and the enzyme is sometimes inactivated in an enzymatic method. Therefore, it is preferable to measure the amount of the fructose after neutralizing the sample.

In this case, a base or an aqueous solution thereof is used to neutralize the hydrolyzed sample, while allowing it to cool to room temperature if necessary and cooling further with ice water in some cases. As examples of the base, there may be mentioned sodium hydroxide, sodium carbonate, sodium hydrogencarbonate, potassium carbonate, potassium hydrogencarbonate, or barium carbonate. When the hydrolysis is performed with sulfuric acid, it is preferable to use solid barium carbonate for neutralization, because it is possible to maintain the neutral range even if a small excess amount of base is added from the neutral point without increasing the volume of the solution. The final pH is preferably 5 to 7.5. If the final pH is less than 5, there is an adverse influence on the liquid chromatography column or inactivation of the enzyme. If the final pH is more than 7.5, the fructose is decomposed while being stored for a long time. Further, it is preferable to use an aqueous solution of potassium carbonate for the neutralization when perchloric acid is used for removing a protein and for hydrolysis.

Step 3: Measurement of amount of fructose

The amount of fructose can be measured by any one of known detecting methods. For example, (A) a method for measurement using high performance liquid chromatography with a pulsed electrochemical detector, (B) an enzymatic method, or (C) the color formation method may be used. In the present invention, it is preferable to use the above method (A) or (B), because it is not necessary to use concentrated sulfuric acid therein, and it is more preferable to use the method (A) because the sensitivity and quantifiability are good.

(A) Method using high performance liquid chromatography with pulsed electrochemical detector:

An assay capable of directly detecting saccharides with high sensitivity using a pulsed electrochemical detector (ECD) [pulsed amperometric detector (PAD)] is developed using high performance liquid

chromatography. In this method, aqueous sodium hydroxide solution is used as a mobile phase, the saccharide is dissociated to an anion, separated by an anion exchange column, and then detected by the pulsed ECD (direct detection-pulsed ECD).

As an improved method, there is developed a method for detecting a saccharide by separating the saccharide by means of a normal phase partition column, ligand exchange column, size exclusion column or cation exchange column as well as anion exchange column, then adding an alkali solution to the mobile phase, such as a sodium hydroxide solution as a reaction solution to raise the pH and dissociate the hydroxyl groups of the saccharide to an anion, and then detecting the anion by a pulsed ECD (postcolumn-pulsed ECD). In this case, an alkali aqueous solution, acetonitrile, borate butter or water is used as the mobile phase (Tomoyoshi Soga, Monthly Food Chemical, December 1991, pp. 44 - 48).

A method for detecting a saccharide by separating the saccharide by high performance liquid chromatography and detecting by a pulsed ECD is known to be applied in the field of food [Tomoyoshi Soga, Monthly Food Chemical, December 1991, pp 44 - 48; D.R. White Jr. et al., J. Agric. Food Chem., Vol. 38, pp. 1918 - 1921 (1990)], but application for inulin clearance is not known hitherto.

Gold electrodes are usually used in the pulsed ECD. When a voltage is constant, oxides are deposited on the electrode surface and the further oxidation of the saccharides is inhibited. Therefore, a measurement potential, a higher potential than the measurement potential (oxidation cleaning), and a negative potential (reduction potential) are successively instantaneously applied to clean the oxides deposited on the electrodes and continue the measurement. That is, the pulsed potential waveform consists of three potentials.

It is possible to use either of a direct detection-pulsed ECD or postcolumn-pulsed ECD in the method of the present invention, but a direct detection-pulsed ECD is preferable in view of high sensitivity. As the mobile phase for the high performance livid chromatography, it is preferable to use 0.05 to 0.3N NaOH, and as the stationary pose it is preferable to use an anion exchange resin.

The preferred pulse cycle of the pulsed ECD is 250 to 1500 ms. The preferred electrode potentials (Ag/AgCl reference; $E_1$, $E_2$, $E_3$) and duration time (T1, T2, T3) are: $E_1$ = 50 to 200mV (T1 = 100 to 500ms), $E_2$ = 400 to 800mV (T2 = 50 to 300ms), and $E_3$ = -200 to -1100mV (T3 = 100 to 700ms).

(B) Enzymatic method

It is possible to measure fructose enzymatically in accordance with the methods described in literatures. For example, there are (a) the method of Kuehnle et al. [Japanese Unexamined Patent Publication (Kokai) No. 62-205799, H.F. Kuehnle et al., Nephron, Vol. 62, pp. 104 - 107 (1992)], (b) the method of Hans-Otto et al. (EP 0021310 A1), and (c) the method of Day et al. (D.F. Day et al., Ann. N.Y. Acad. Sci. (USA), Vol. 434, pp. 504 - 507 (1984)].

(a) Method of Kuehnle et al.

Fructose is phosphorylated with adenosine 5'-triphosphate (ATP) and hexokinase (HK), the resulting fructose-6-phosphate is isomerized with glucose-6-phosphate isomerase (PGI) to glucose-6-phosphate, and the resulting glucose-6-phosphate is converted by nicotinamide adenine dinucleotide phosphate (NADP) and glucose-6-phosphate-dehydrogenase (G6PDH) to gluconate-6-phosphate and NADPH (reduced NADP). The quantity of fructose is measured by spectroscopical measurement of the amount of NADP converted to NADPH.

(b) Method of Hans-Otto et al.

Fructose is reacted with uridine 5'-diphosphoglucose (UDPG) and saccharose synthetase to form uridine 5'-diphosphate (UDP) and the resulting UDP is measured by a known method. For example, UDP is reacted with phosphoenolpyruvate (PEP) in the presence of pyruvate kinase (PK), lactate dehydrogenase (LDH) and reduced form of nicotineamide adenine dinucleotide (NADH) to produce lactate and nicotineamide adenine dinucleotide (NAD). The quantity of fructose is measured by spectroscopical measurement of the amount of NADH converted to NAD.

(c) Method of Day et al.

Fructose is reacted with sorbitol dehydrogenase (SDH) in the presence of NADH to produce sorbitol and NAD. The quantity of fructose is measured by spectroscopical measurement of the amount of NADH

converted to NAD.

(C) Color formation method:

It is possible to measure the amount of fructose in accordance with the color formation method described in literatures. This method comprises reacting fructose with one of various coloring agents and spectroscopical measurement of the amount of fructose. For the color formation, methods of treatment with diphenylamine [H.D. Harrison, Proc. Soc. Exp. Biol. Med., Vol. 49, pp. 111 - 114 (1942)], resorcin [G.F. Schreiner, Proc. Soc. Exp. Biol. Med., Vol. 74, pp. 117 - 120 (1950)], or anthrone [W.D. Davidson et al., J. Lab. Clin. Med., Vol 62, pp. 351 - 356 (1963)] are known.

When the kidney function is lowered, the inulin clearance is reduced. The method for the assay of inulin according to the present invention can be used for diagnosis of various diseases in which the kidney function is lowered, such as kidney diseases or other abnormalities. Examples of the various diseases and abnormalities causing a decline in the inulin clearance are acute renal insufficiency, chronic renal insufficiency, nephrosclerosis (hypertension), pyelitis, acute glomerulonephritis, chronic glomerulonephritis, multiple myeloma, sodium deficiency, shock, bleeding, norepinephrine administration or nocturia.

The measurement per se of inulin clearance may be performed using conventionally known methods (Yasuhiko Iino et al., Nihon Rinsho, Vol. 47, 1989 Supplement, pp, 443 - 446). For example, a catheter is inserted to the patient's vein for the continuous administration of inulin until a stable concentration is reached, and blood and urine are taken at predetermined intervals. The plasma is immediately separated from the blood. The inulin concentrations in the plasma and urine sample are measured by the method of the present invention and the resulting values are used to calculate the inulin clearance by a known formula.

The method for the assay of inulin according to the present invention may be applied not only to the conventional method for determining inulin clearance wherein inulin is continuously injected, but also to the method for determining inulin clearance by calculation from elimination rate of inulin administered by a single dosage of inulin.

The inulin used for the determination of the inulin clearance is not especially limited, but for example may be those isolated from natural materials, such as tubers of dahlia, Helianthus tuberosus, and Chicorium intybus, stems of sea onion, or seaweed belonging to the Acetabularia genus or those commercially available as a reagent. Further Inutest (trade name) (polyfructosan) may be used in the same manner as inulin. Inutest is available from Laevosan GmbH as Inutest-Ampullen (containing 5.0 g of polyfructosan).

According to the method for the assay of inulin of the present invention, inulin in a sample can be accurately determined to approximately 1/100th the concentration in the conventional anthrone method when using HPLC-ECD method, and further, approximately 1/5th the concentration in the conventional enzymatic method when using enzymatic method.

## Examples

The present invention now will be further illustrated by, but is by no means limited to, the following Examples

### Example 1: Calibration curve in inulin microanalysis

An aqueous solution of inulin (Wako Pure Chemical Industries) (1 ml) was added to the pooled plasma (1 ml) of renal insufficiency patients to prepare samples with an inulin concentration of 10.0 mg/dl, 5.0 mg/dl, 2.5 mg/dl and 1.25 mg/dl (hereinafter referred to as the samples A). Further, inulin was added to 0.45 % saline solution to prepare samples with an inulin concentration of 10.0 mg/dl, 5.0 mg/dl, 2.5 mg/dl and 1.25 mg/dl (hereinafter referred to as the samples B). The inulin used was the reagent from Wako Pure Chemical Industries.

The concentration of inulin was determined by the method of the present invention for each of the above samples. The method of measurement was as follows:

(1) Dialysis

Samples (1.5 ml) were placed in a flow type dialyzer (cell capacity = 2 ml; 10 cells). 0.45 % saline solution (2 liters) as a dialysis solution was used, and dialysis was performed at a circulation flow rate of 20 ml/min at room temperature for 20 hours. The dialysis membrane used was a regenerated cellulose membrane rated to retain substances of molecular weight of more than 1000 (regenerated cellulose dialysis

tube: Spetra/Pore 6; 38 mm wide; Spectrum Medical Industries, Inc.)

(2) Protein removal

5 % trichloroacetic acid (1 ml) was added to a dialysed sample (1 ml) of the sample A. The supernatant free of proteins was obtained by centrifugal separation.

(3) Acid-hydrolysis

To the supernatant (1 ml) free of proteins or a mixture of the sample B (0.5 ml) and 5% trichloroacetic acid (0.5 ml), 12N sulfuric acid (50 µl) was added (final concentration = about 1N) to perform hydrolysis at 60 °C for 5 hours. Solid barium carbonate was added to neutralize hydrolyzed solution.

(4) Measurement of quantity of fructose by high performance liquid chromatography with pulsed electrochemical detector

A neutralized sample (50 µl) was injected into a high performance liquid chromatograph/pulsed electrochemical detection system (BIO LC made by DIONEX; $E_1$ = 100mV, T1 = 300ms, $E_2$ = 600mV, T2 = 120ms, $E_3$ = - 800mV, T3 = 300ms, pulse cycle = 720 ms). An anion exchange column was used.

The resulting inulin calibration curve is shown in Fig. 1. The abscissa is the concentration of the inulin solution and the ordinate is the peak area. Linearity was observed in the range of an inulin solution concentration of 1.25 to 10 mg/dl. Almost the same lines were obtained for the samples A and B. Therefore, it is manifest that the assay is not affected by the proteins and saccharides such as glucose existing in the plasma.

Example 2: Comparison of the method of present invention and anthrone method

In the present Example, quantification by high performance liquid chromatography with a pulsed electrochemical detector was used for the determination of fructose in the method of the present invention.

An aqueous solution of inulin (0.1 ml) was added to the pooled plasma (2.9 ml) from patients suffering from renal insufficiency to prepare samples of different concentrations (9 kinds). The inulin used was the reagent of Wako Pure chemical Industries. A comparison was made of the method of the present invention and the conventional anthrone method for these samples.

(1) Measurement of inulin by the method of the present invention

Samples of inulin in a concentration of 30.0 mg/dl, 15.0 mg/dl, 7.0 mg/dl, 4.0 mg/dl, 3.0 mg/dl, 1.5 mg/dl, 0.7 mg/dl, 0.3 mg/dl and 0.15 mg/dl were prepared and the amount of the inulin was measured in the same manner as in Example 1.

(2) Measurement of inulin by anthrone method

The measurement was performed in accordance with the method described in Yasuyoshi Mito, Nihon Rinsho, Vol. 37, summer Supplement, pp. 1283 - 1284.

Samples of inulin in a concentration of 40.0 mg/dl, 30.0 mg/dl, 15.0 mg/dl, 7.0 mg/dl, 3.0 mg/dl, 1.5 mg/dl, 0.7 mg/dl, 0.3 mg/dl and 0.15 mg/dl were prepared.

5 % trichloroacetic acid (200 µl) was added to each of the samples (200 µl) and the proteins were removed by centrifugal separation. The resulting supernatant was used as the specimen.

The anthrone reagent was prepared as follows: Concentrated sulfuric acid (250 ml) was mixed with distilled water (100 ml) and the mixture was placed in a refrigerator overnight. On the next day, anthrone (obtained from Kishida chemical: 0.7 g) was added to the sulfuric acid solution and the anthrone was completely dissolved in a constant-temperature bath of 56 °C. The resulting yellow transparent solution which can be used was placed in a brown bottle and stored in a refrigerator.

The specimen (0.05 ml) was added to 5 ml of the anthrone reagent and thoroughly mixed. The mixture was incubated at 37 °C for 50 minutes, and then a photoelectric colorimeter (Shimazu UV-160A) was used to measure the absorbance at 636 nm.

The results are shown in Fig. 2 in logarithmic scale. The abscissa indicates the inulin concentration (mg/dl) in the sample, while the ordinate indicates the peak area by the pulsed electrochemical detector in

the method of the present invention and the absorbance by a photoelectric colorimeter in the anthrone method.

In the anthrone method, there was observed a large deviation from the straight line at an inulin concentration of less than 15 mg/dl. The result coincides with the fact that the most reliably quantifiable concentration in the anthrone method is considered to be around 25 mg/dl (plasma). Whereas, a good linearity was observed in the method of the present invention as low as 0.15 mg/dl. The fact shows that the method of the present invention is able to precisely measure to an inulin concentration of about 1/100th that of the conventional anthrone method.

Example 3: Comparison of the method of the present invention and conventional enzymatic method

In the present Example, an enzymatic method for the determination of fructose is used in the method of the present invention.

To the pooled plasma (2.9 ml) from patients suffering from renal insufficiency, an aqueous solution of inulin (0.1 ml) was added to prepare samples (hereinafter referred to as samples C) of inulin in a concentration of 20.0 mg/dl, 10.0 mg/dl, 5.0 mg/dl, 1.0 mg/dl and 0.5 mg/dl. The inulin used was the reagent of Wako Pure Chemical Industries.

(1) Measurement of inulin by the method of the present invention

A sample C was dialyzed in the same manner as in Example 1 (1). The dialyzed sample (1 ml) was transferred to a centrifugation tube and perchloric acid (0.6M; 1 ml) was added and thoroughly mixed. The mixture was warmed in a warm bath (80 °C) for 15 minutes, and centrifuged after cooling to room temperature to remove the proteins and perform the acid-hydrolysis. To the supernatant (1.5 ml), an aqueous solution of potassium carbonate (0.75M; 0.5 ml) was added. The whole was mixed to neutralize the supernatant. The mixture was cooled with ice for 15 minutes, and centrifuged. The supernatant (1 ml) (hereinafter referred to as the sample C1) was used for the assay of fructose by the enzymatic method described in the following item (3).

(2) Measurement of inulin by conventional enzymatic method

The sample C (0.5 ml) and perchloric acid (0.6M; 0.5 ml) were added to a centrifugation tube and mixed. The mixture was warmed in a warm bath (80 °C) for 15 minutes, then cooled to room temperature and centrifuged. To the supernatant (0.5 ml), an aqueous solution of potassium carbonate (0.75M; 0.2 ml) was added. The whole was mixed to neutralize the supernatant. The mixture was cooled with ice for 15 minutes, and then centrifuged. The supernatant (0.25 ml) (hereinafter referred to as the sample C2) was used for the assay of fructose by the enzyme method explained in the following (3).

(3) Assay of fructose by enzymatic method

As the assay agent for fructose by the enzymatic method, the F-kit: glucose/fructose (made by Boeringer Mannheim) was used. The principle of measurement of the above reagent is as follows:

$$(1)\quad \text{Glucose} + \text{ATP} \xrightarrow{\text{HK}} \text{glucose-6-phosphate} + \text{ADP}$$

$$(2)\quad \text{Fructose} + \text{ATP} \xrightarrow{\text{HK}} \text{fructose-6-phosphate} + \text{ADP}$$

(3) Glucose-6-phosphate + $NADP^+$ --G6PDH--> gluconate-6-phosphate + NADPH + $H^+$

(4) Fructose-6-phosphate <--PGI--> glucose-6-phosphate

In the above formulas, ATP is adenosine 5'-triphosphate, HK is hexokinase, ADP is adenosine 5'-diphosphate, NADP is nicotineamide adenine dinucleotide phosphate, G6PDH is glucose-6-phosphate dehydrogenase, NADPH is a reduced form of nicotineamide adenine dinucleotide phosphate, and PGI is phosphoglucose-6-isomerase.

The amount of NADPH formed in the reaction of the formulas (1) and (3) is quantitative with the amount of glucose, and thus the absorbance at 340 nm was measured.

More particularly, first, the reactions of formulas (1), (2) and (3) were performed, and the amount of glucose was measured (absorbance $E_1$). Then, PGI was added, the reactions of formulas (4) and (3) were performed, the total amount of glucose and fructose was measured (absorbance $E_2$), and the amount or fructose was calucuated as $E_2$-$E_1$. Further, the ($E_2$-$E_1$) obtained by a blank test was subtracted to make correction.

The following solutions 1, 2 and 3 were used for the fructose assay:

Solution 1: Triethanolamine buffer (pH 7.6), NADP (64 mg), ATP (160 mg) $MgSO_4$ and a stabilizer (dissolved in 27 ml of distilled water)

Solution 2: HK (approximately 200 U) and G6PDH (approximately 100 U) (solution volume = 0.7 ml)

Solution 3: PGI (approximately 490 U) (solution volume = 0.7 ml)

The method of the assay of fructose was as follows:

As a sample, the sample C1 (1 ml) or sample C2 (0.25 ml) was used. A sample blank prepared by treating in the same manner as in the preparation of the sample C1 or C2 plasma without an aqueous solution of inulin was used in the same amount as the samples.

The solution 1 (1.00 ml), a sample or sample blank, distilled water (in an amount to 2.0 ml together with the sample or sample blank), and the solution 2 (0.02 ml) were transferred into a cuvette (optical path length of 1 cm) and mixed. Then, the whole was allowed to stand and perform a reaction for about 15 minutes. Then, a photoelectric colorimeter (Shimazu UV-160A) was used to measure the absorbance ($E_1$) at 20 to 25°C and 340 nm using water as a control.

Then, the solution 3 (0.02 ml) was added and the whole was mixed. Then, the mixture was allowed to stand and perform a reaction for approximately 15 minutes, and the absorbance ($E_2$) was measured in the same manner as above.

The difference of absorbance ($E_2$-$E_1$) of the sample blank was subtracted from the difference of absorbance ($E_2$-$E_1$) of the sample to obtain the absorbance (ΔE) corresponding to fructose.

The results of the method of the present invention are shown in Table 1, whereas the results of the conventional enzymatic method are shown in Table 2. Further, the $E_2$ values are plotted with regard to the concentration of inulin in plasma and are shown in Fig. 3.

Table 1

| Method of Present Invention | | | |
|---|---|---|---|
| Inulin concentration in sample mg/dl | Absorbance | | Fructose absorbance ΔE |
| | $E_1$ | $E_2$ | |
| 0.5 | 0.141 | 0.155 | 0.010 |
| 1.0 | 0.142 | 0.169 | 0.023 |
| 2.5 | 0.138 | 0.185 | 0.043 |
| 5.0 | 0.151 | 0.242 | 0.087 |
| 10.0 | 0.147 | 0.313 | 0.162 |
| 20.0 | 0.145 | 0.498 | 0.349 |
| Sample blank: $E_1$ = 0.141, $E_2$ = 0.145 ($E_2$-$E_1$ ) = 0.004 | | | |

Table 2

| Conventional Enzymatic Method | | | |
|---|---|---|---|
| Inulin concentration in sample mg/dl | Absorbance | | Fructose absorbance ΔE |
| | $E_1$ | $E_2$ | |
| 0.5 | 1.024 | 1.033 | 0.006 |
| 1.0 | 1.088 | 1.104 | 0.013 |
| 2.5 | 1.078 | 1.106 | 0.025 |
| 5.0 | 1.087 | 1.140 | 0.050 |
| 10.0 | 1.026 | 1.120 | 0.091 |
| 20.0 | 1.074 | 1.273 | 0.196 |
| Sample blank: $E_1$ = 1.063, $E_2$ = 1.066 ($E_2$-$E_1$) = 0.003 | | | |

In the conventional enzymatic method, the $E_1$ value is high due to a large amount of glucose present in the plasma. On the contrary, in the method of the present invention, the $E_1$ value falls to about 1/10th although a double amount of plasma was used. This advantage was brought about by dialysis. Accordingly, it is possible to increase the amount of the sample and measure a low concentration of inulin, using a dialyzed sample. Further, because the background due to the interferents such as glucose becomes lower, the precision of the assay is improved. This advantage is clear from Fig. 3. More particularly, the absorbance $E_2$ corresponding to the total amount of the glucose and fructose becomes a high value close to approximately 1.5 which is the limit of measurement of the absorbance, in the case of the sample without dialysis. The deviation from the straight line is large as well. On the contrary, in the case of a dialyzed sample, the absorbance $E_2$ value was low and in the measurable region, although a double amount of plasma was used. Further, the results show that a measurement can effectively be performed even if the amount of the sample is increased, and that a precision measurement with little variation is possible.

It is assumed from Fig. 3 that about 5 times amount of the plasma may be used in the method of the present invention. This means that an accurate measurement can be effected to a concentration of about 1/5th that of the conventional enzymatic method, according to the present invention.

As above, the present invention was explained with reference to particular embodiments, but modifications and improvements obvious to those skilled in the art are included in the scope of the present invention.

## Claims

**1.** A method for the assay of inulin in a sample comprising the steps of:
(1) removing from the sample, glucose and fructose through a membrane; then

(2) treating the sample obtained from the above step 1 under acid-hydrolysis conditions capable of hydrolyzing inulin to fructose; and then
(3) measuring the amount of fructose in the sample obtained from the above step 2.

**2.** A method according to claim 1, wherein the step 2 is carried out after removing a protein from the sample obtained from the step 1.

**3.** A method according to claim 1, wherein a dialysis membrane is used in the step 1.

**4.** A method according to claim 3, wherein the dialysis membrane capable of removing a substance having a molecular weight of not more than 1000 is used.

**5.** A method according to claim 1, wherein the sample obtained from the step 2 is neutralized.

**6.** A method according to claim 1, wherein quantification by high performance liquid chromatography with a pulsed electrochemical detector are used to determine fructose in the step 3.

**7.** A method according to claim 1, wherein an enzymatic method is used to determine fructose in the step 3.

**8.** A method according to claim 1, wherein 2 to 15 % trichloroacetic acid is used for removal or a protein, 0.5 to 2.0N sulfuric acid is used for acid-hydrolysis, and then solid barium carbonate is used for neutralization between the step 1 and step 3.

**9.** A method according to claim 1, wherein 0.1 to 2.0N perchloric acid is used for removal of a protein, the resulting sample free of proteins is heated to 20 to 100°C to perform acid-hydrolysis, and then potassium carbonate aqueous solution is used for neutralization between the step 1 and step 3.

**10.** A method according to claim 1, wherein the sample is plasma, serum or urine, or a dilution thereof with water, saline, physiological saline, or a buffer.

**11.** A method for determining inulin clearance by the method for the assay of inulin according to claim 1.

**12.** A method for the diagnosis of a disease lowering a kidney function by the method for the assay of inulin according to claim 1.

Fig. 1

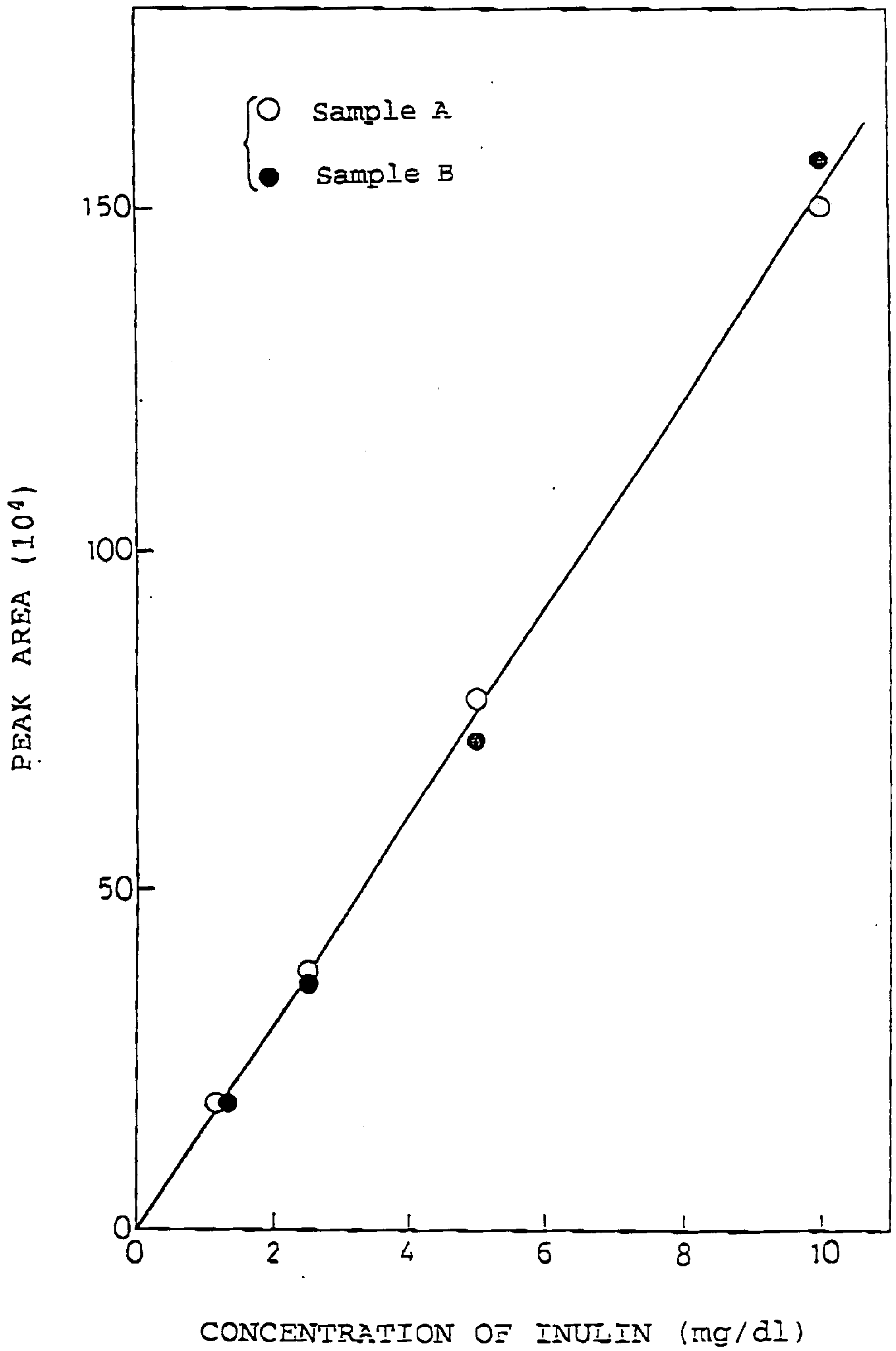
Sample A
Sample B
PEAK AREA ($10^4$)
150
100
50
0
0
2
4
6
8
10
CONCENTRATION OF INULIN (mg/dl)

Fig. 2

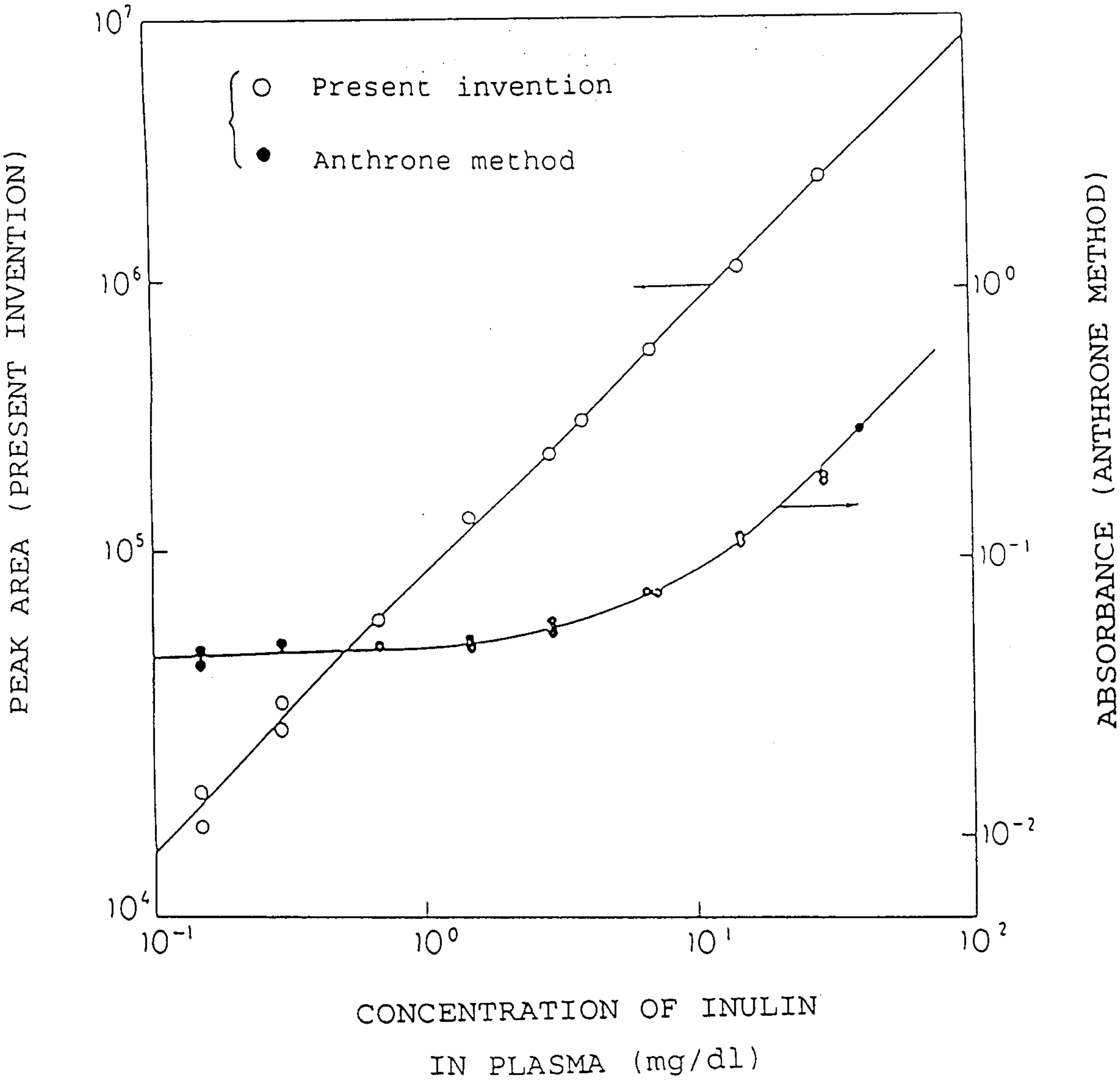

Present invention
Anthrone method
PEAK AREA (PRESENT INVENTION)
ABSORBANCE (ANTHRONE METHOD)
$10^7$
$10^6$
$10^5$
$10^4$
$10^0$
$10^{-1}$
$10^{-2}$
$10^{-1}$
$10^0$
$10^1$
$10^2$
CONCENTRATION OF INULIN
IN PLASMA (mg/dl)

Fig. 3

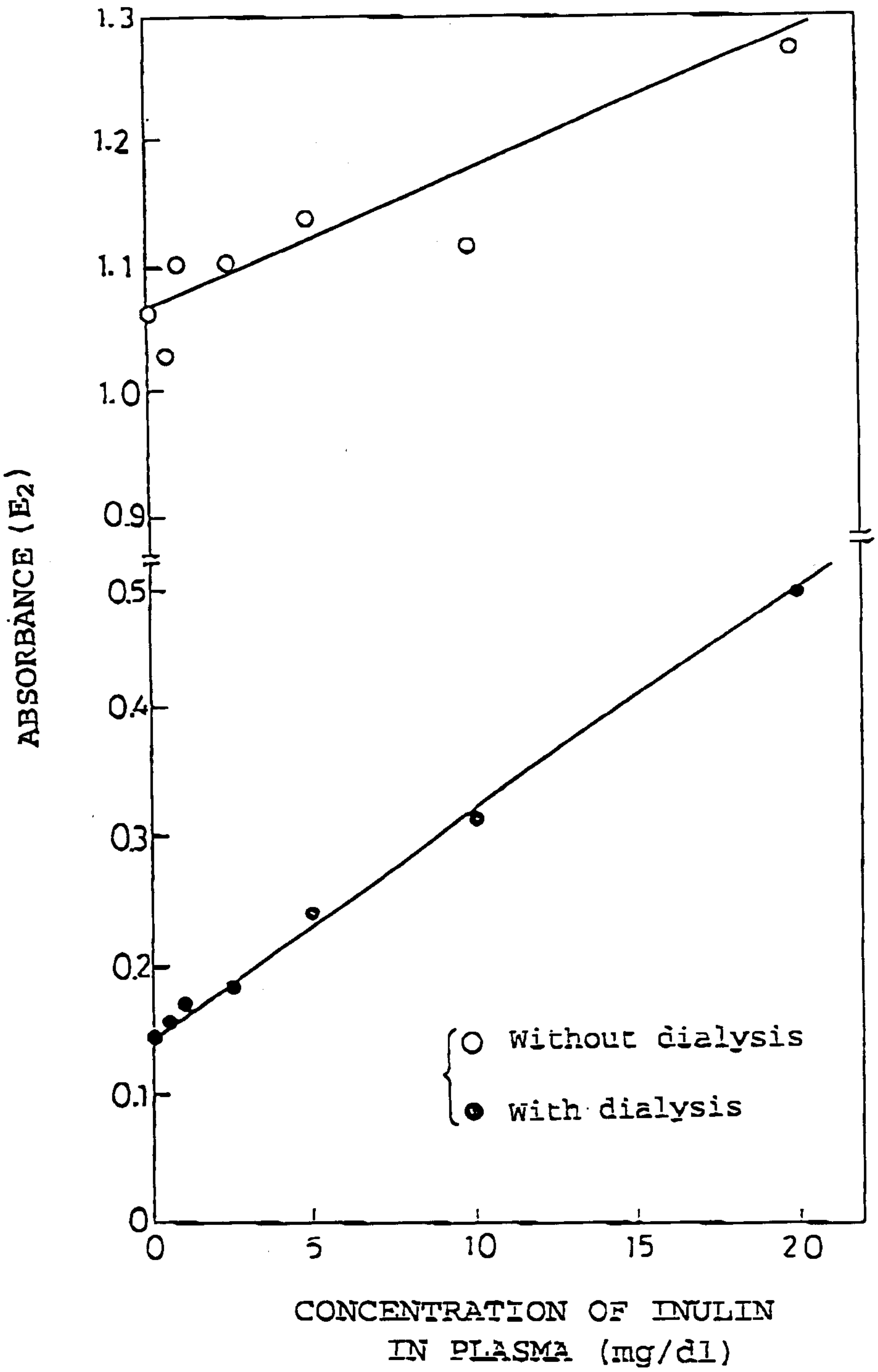
1.3
1.2
1.1
1.0
0.9
0.5
0.4
0.3
0.2
0.1
0
ABSORBANCE ($E_2$)
0
5
10
15
20
CONCENTRATION OF INULIN IN PLASMA (mg/dl)
Without dialysis
With dialysis

European Patent Office

# EUROPEAN SEARCH REPORT

Application Number EP 95102235.9

| Category | Citation of document with indication, where appropriate, of relevant passages | Relevant to claim | CLASSIFICATION OF THE APPLICATION (Int. Cl. 6) |
|---|---|---|---|
| D,A | ANALYTICAL BIOCHEMISTRY, An International Journal of Analytical and Preparative Methods, vol. 94, 1979 P.H. BRAND et al. "Improved Microdialysis Technique" pages 109-111 * Page 110-111, discussion * -- | 1,3,4 | C 07 H 1/06 C 12 Q 1/54 //A 61 K 49/00 |
| A | DE - A - 3 605 572 (BOEHRINGER MANNHEIM GMBH) * Claims 1-5 * -- | 1,7, 12 | |
| D,A | EP - A - 0 021 310 (BOEHRINGER MANNHEIM GMBH) * Abstract; page 4, lines 16-21 * ---- | 1,7, 12 | |
| | | | TECHNICAL FIELDS SEARCHED (Int. Cl.6) |
| | | | C 07 H C 12 Q A 61 K |

The present search report has been drawn up for all claims

| Place of search | Date of completion of the search | Examiner |
|---|---|---|
| VIENNA | 03-05-1995 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)